# EUROPEAN PATENT APPLICATION

(11) **EP 1 022 269 A2**
(43) Date of publication of application: **26.07.2000**
(21) Application number: 99305297.6
(22) Date of filing: 02.07.1999
(51) Int. Cl.: C07C 381/12, B01J 27/02

(54) **Sulfonium salts and method of synthesis**

(30) Priority: 19.01.1999 KR 9901494
(71) Applicant: Korea Kumho Petrochemical Co. Ltd., Chongno-ku, Seoul 110-110 (KR)
(72) Inventor: Park, Joo Hyeon, Yousung-ku, Taejeon-city 305-390 (KR); Lee, Jong Bum, Yousung-ku, Taejeon-city 305-345 (KR); Kim, Jae Young, Yousung-ku, Taejeon-city 305-345 (KR)
(74) Representative: Nash, David Allan

(57) **Abstract**

This invention relates to a process of synthesizing a novel sulfonium salt represented by the following formula 1 in such a manner that the sulfide compound represented by the following formula 2 is reacted with perfluoroalkanesulfonic anhydride represented by the following formula 3:

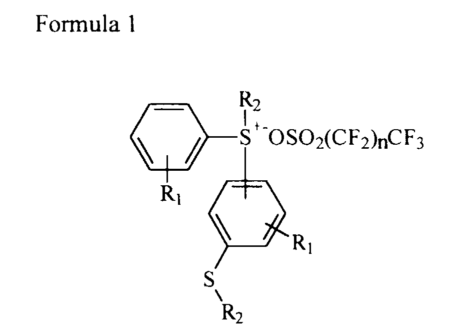

Wherein, R₁ is a hydrogen atom, an alkyl group of 1 to 20 carbon atoms in a linear, branched or cyclic form, halogen atom, alkoxy group, phenoxy group, phenyl group, thioalkoxy group, or thiophenoxy group;
R₂ is an alkyl group of 1 to 20 carbon atoms in a linear, branched or cyclic form, aryl group or allyl group;
n is an integer of 0 to 20.

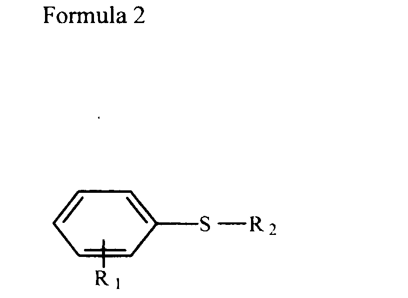

Wherein, R₁ and R₂ are same as defined above.

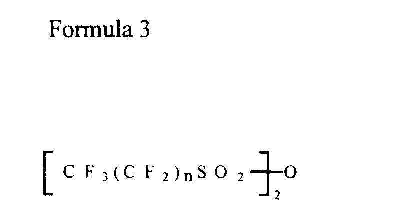

Wherein, n is same as defined above.

The process of manufacturing a novel sulfonium salt according to this invention can provide the following advantages in that a) sulfonium salt can be synthesized via one step reaction using perfluoroalkanesulfonic anhydride, b) sulfonium salt having higher yield than high-priced sulfoxide compound can be also synthesized using low-priced sulfide compound. Further, sulfonium salt is effectively used as a photo-acid initiator or radical photo-initiator in polymerisation and as a catalyst to leave the protective groups of organic compounds, especially as a photo-acid generator in a chemical amplified photoresist used for semiconductor material.

## Description

### Field of the Invention

This invention relates to a synthesis of novel sulfonium salts and synthesizing method thereof and more particularly, to sulfonium salts represented by the following formula 1, being effectively used as a photo-acid initiator or radical photo-initiator in polymerization and as a photo-acid generator in a chemical amplified photoresist, and its synthetic method thereof. 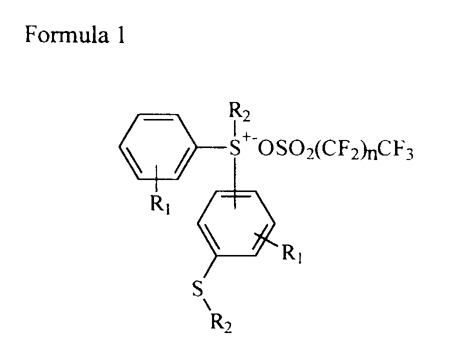
Wherein, R₁ is a hydrogen atom, an alkyl group of 1 to 20 carbon atoms in a linear, branched or cyclic form, halogen atom, alkoxy group, phenoxy group, phenyl group, thioalkoxy group, or thiophenoxy group;
R₂ is an alkyl group of 1 to 20 carbon atoms in a linear, branched or cyclic form, aryl group or allyl group;
n is an integer of 0 to 20.

### Description of the Related Art

In general, sulfonium salts have been used as a photo-acid initiator or radical photo-initiator during polymerization, or as an acid catalyst generator to promote the deprotection of organic compounds. Recently, it is widely employed as a photo-acid generator in a chemical amplified photoresist.

The conventional method to synthesize sulfonium salts with such functions was disclosed by using a sulfoxide compound and Grignard reagent in two reaction steps (EP 0 327 194 A1). However, this method has recognized some disadvantages for commercial production in that a) some high-priced sulfoxide compound should be employed, b) two reaction steps should be necessary, and c) yield is poor.

To comply with these matters, another method to synthesize sulfonium salt via phosphorus pentoxide and methanesulfonic acid was developed (EP 0 455 083 A1) but this synthesis method has still failed to improve the conventional drawbacks that some high-priced sulfoxide compound should be employed and two reaction steps should be necessary.

### SUMMARY OF THE INVENTION

An object of this invention is to provide a novel sulfonium salts.

Further object of this invention is to provide a process of synthhesizing sulfonium salts in such a manner that unlike the conventional method, alkyl aryl sulfide or diaryl sulfide is used as a starting material instead of high-priced sulfoxide and a final product can be obtained with a high yield via one reaction step in the absence of Grignard reagent.

### Detailed Description of the Invention

To achieve this objective, sulfonium salt of this invention is characterized by the expression of the following formula 1. 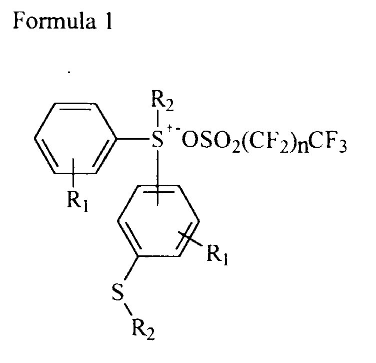
Wherein, R₁ is a hydrogen atom, an alkyl group of 1 to 20 carbon atoms in a linear, branched or cyclic form, halogen atom, alkoxy group, phenoxy group, phenyl group, thioalkoxy group, or thiophenoxy group;
R₂ is an alkyl group of 1 to 20 carbon atoms in a linear, branched or cyclic form, aryl group or allyl group;
n is an integer of 0 to 20.

However, this invetion relates to a process of synthesizing the sulfonium salts represented by the above formula 1 in such a manner that the sulfide compound represented by the following formula 2 is reacted with perfluoroalkanesulfonic anhydride represented by the following formula 3.

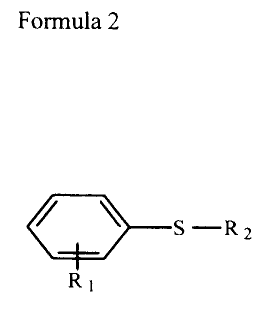

Wherein, R₁ and R₂ are same as defined above.

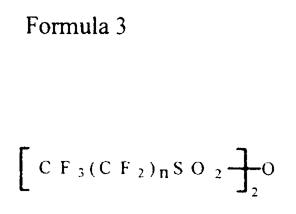

Wherein, n is same as defined above.

This invention is explained in more detail as set forth hereunder.

According to this invention, the process of manufacturing sulfonium salt is one step reaction, wherein 1 equivalent of perfluoroalkanesulfonic anhydride is added to 2 equivalents of sulfide compound dissolved in solvent and stirred.

According to this invention, the process of manufacturing sulfonium salt can be represented by the following scheme 1.

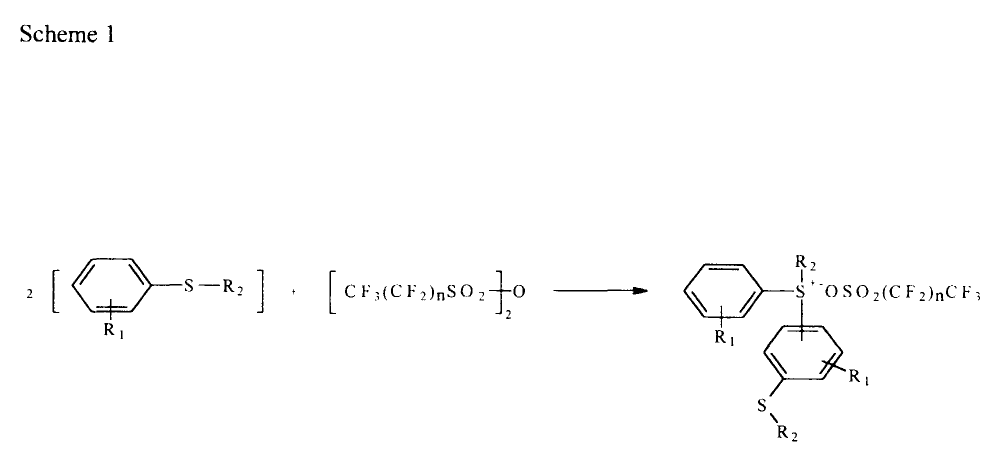

Wherein R₁, R₂ and n are same as defined above.

As defined above, the sulfide compound represented by the formula 2, which is used for the manufacture of sulfonium salt of this invention may be selected from the commonly used sulfide compounds such as diaryl sulfide derivative, alkyl aryl sulfide derivative and allyl aryl sulfide derivative.

Among these sulfide compounds, however, the reactability of diaryl sulfide derivative is lower than alkyl aryl sulfide derivative or allyl aryl sulfide derivative.

Further, perfluoroalkanesulfonic anhydride represented by the formula 3 may contain all of the commonly used compounds and detailed examples of perfluoroalkanesulfonic anhydride include trifluoromethane sulfonic anhydride, perfluorobutane sulfonic anhydride, and perfluorooctanesulfonic anhydride.

The amount of perfluoroalkanesulfonic anhydride used for this invention in the range of 0.2 to 2.0 equivalents in proportion to sulfide derivative represented by the formula 2, more preferably in the range of 0.4 to 0.75 equivalents. If the amount of perfluoroalkanesulfonic anhydride represented by the formula 3 is less than 0.2 equivalents in proportion to the sulfide derivative, yield increased but sulfide derivative wastes unnecessarily, and if exceeding 2.0 equivalents, yield is poor.

In line with a process of manufacturing sulfonium salt using these compounds, reaction solvents (e.g., dichloromethane, chloroform, carbontetrachloride, ether, pentane, hexane, benzene and toluene) are not specifically confined. If the situation permits, any reaction solvent is unnecessary.

Further, sulfonium salt is prepared at -100∼100°C. If the reaction temperature is high, some by-products may be generated and this results in reducing the yield of a final product.

When the patterns of a photoresist composition comprising sulfonium salt as acid generator with the addition of base resin and a small amount of additives are investigated, more ultrafine patterns may be formed compared with those using the conventional sulfonium salt. Also, sulfonium salt of this invention is effectively used as a photo-acid initiator or radical photo-initiator in polymerization and as a catalyst to leave the protecting groups of organic compounds.

This invention is explained in more detail based on the following Examples but is not limited by these Examples.

### Example 1: Synthesis of methyl(4-thiomethoxyphenyl)phenylsulfonium triflate

10g of thioanisole was dissolved in 50ml dichloromethane and while cooling the internal temperature of a reactor to less than -10°C, 10.3g of triflic anhydride was slowly dropwise added to the mixture. After the addition was completed, the reacting mixture was stirred at the same temperature for 30 minutes and then, the temperature was slowly up to room temperature. The reacting mixture was stirred at room temperature for 1 hour and washed with distilled water. After evaporating a solvent from the organic layer, so washed, an oily phase with a larger viscosity was obtained. The oily phase was dissolved in dichloromethane and with the addition of excess of ether, a white crystalline precipitate was obtained. The precipitate was filtered off and evaporated under vacuum to obtain methyl(4-thiomethoxyphenyl)phenylsulfonium triflate 13.2g(yield: 91%) in white solid represented by the following formula 4.
m.p.: 76∼77°C

¹H-NMR(solvent: CDCI₃): 3 proton peaks at 2.42ppm; 3 proton peaks at 3.68ppm; 2 proton peaks at 7.39ppm; 3 proton peaks at 7.73ppm; 4 proton peaks at 7.92ppm.

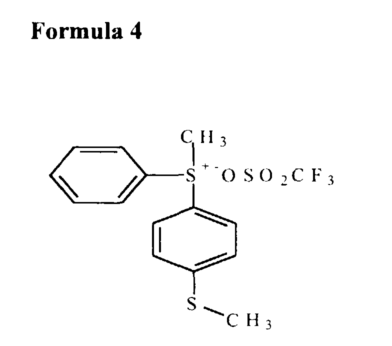

### Example 2: Synthesis of diphenyl(4-thiophenoxy)sulfonium triflate

11.2g of diphenyl(4-thiophenoxy)sulfonium triflate (yield: 88%) represented by the following formula 5 was obtained in the same procedure as Example 1, except that 10g of phenylsulfide and 6.9g of triflic anhydride were used instead of 10g of thioanisole and 10.3g of triflic anhydride, respectively.
m.p.: 81∼82°C

¹H-NMR(solvent: CDCl₃): 3 proton peaks at 7.27ppm; 7 proton peaks at 7.42∼7.59ppm; 10 proton peaks at 8.02ppm.

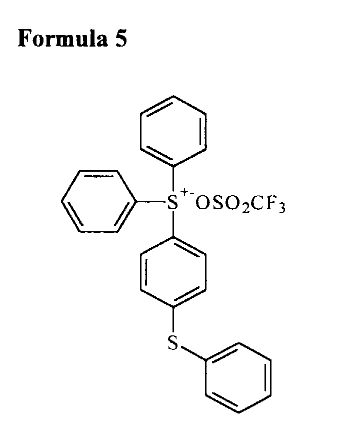

### Example 3: Synthesis of (2-ethyl-hexyl)4-(2-ethyl-hexylsulfanyl)phenylphenylsulfonium triflate

lOg of 4-(2-ethylhexylsulfanyl) benzene dissolved in 50ml dichloromethane and while cooling the internal temperature of a reactor to less than -10°C, 5.8g of triflic anhydride was slowly dropwise added to the mixture. After the addition was completed, the reacting mixture was stirred at the same temperature for 30 minutes and then, the temperature was slowly up to room temperature. The reacting mixture was stirred at room temperature for 1 hour and washed with distilled water. After evaporating a solvent from the organic layer, so washed, an oily phase with a larger viscosity was obtained. Excess of 4-(2-ethylhexylsulfanyl) benzene from the oily phase washed with ether and hexane to obtain (2-ethyl-hexyl)4-(2-ethylhexylsulfanyl)phenylphenylsulfonium triflate in oily phase represented by the following formula 6.

¹H-NMR(solvent: CDCl₃): 12 proton peaks at 0.85ppm; 18 proton peaks at 1.18∼1.79ppm; 2 proton peaks at 2.95ppm; 2 proton peaks at 4.12ppm; 2 proton peaks at 7.43; 3 proton peaks at 7.65ppm; 4 proton peaks at 8.10ppm.

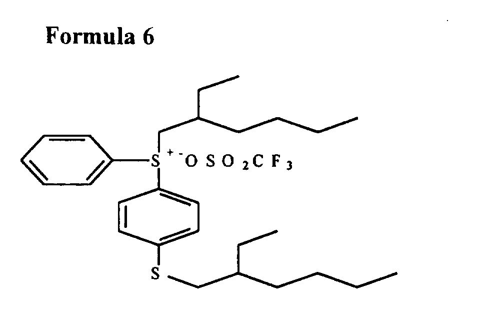

### Example 4: Synthesis of (hexyl)(4-hexylsulfanylphenyl)phenylsulfonium triflate

11.6g of (hexyl)(4-hexylsulfanylphenyl)phenylsulfonium triflate (yield: 93%) represented by the following formula 7 was obtained in the same procedure as Example 3, except that 10g of hexylsulfanyl benzene and 6.6g of triflic anhydride were used instead of 10g of 4-(2-ethylhexylsulfanyl)benzene and 5.8g of triflic anhydride, respectively.

¹H-NMR(solvent: CDCl₃): 6 proton peaks at 0.85ppm; 16 proton peaks at 1.18∼1.80ppm; 2 proton peaks at 2.95ppm; 2 proton peaks at 4.18ppm; 2 proton peaks at 7.41ppm; 3 proton peaks at 7.62ppm; 4 proton peaks at 7.98ppm.

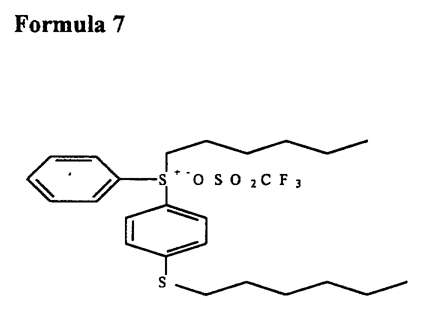

As described above in more detail, this invention can provide the following advantages in that a) sulfonium salt can be synthesized via one step reaction using perfluoroalkanesulfonic anhydride, b) sulfonium salt having higher yield than high-priced sulfoxide compound can be also synthesized using low-priced sulfide compound. Further, sulfonium salt is effectively used as a photo-acid initiator or radical photo-initiator in polymerization and as a catalyst to leave the protective groups of organic compounds, especially as a photo-acid generator in a chemical amplified photoresist used for semiconductor material.

## Claims

1. Sulfonium salt represented by the following formula 1: 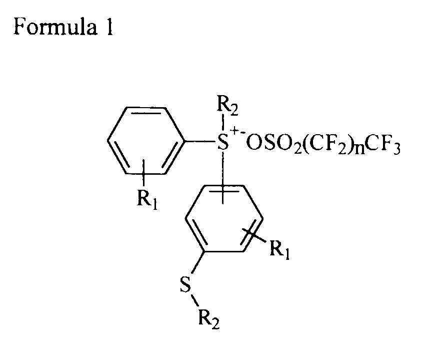
Wherein, R₁ is a hydrogen atom, an alkyl group of 1 to 20 carbon atoms in a linear, branched or cyclic form, halogen atom, alkoxy group, phenoxy group, phenyl group, thioalkoxy group, or thiophenoxy group;
R₂ is an alkyl group of 1 to 20 carbon atoms in a linear, branched or cyclic form, aryl group or allyl group;
n is an integer of 0 to 20.

2. Sulfonium salt according to claim 1, wherein sulfonium salt is selected from the group consisting of

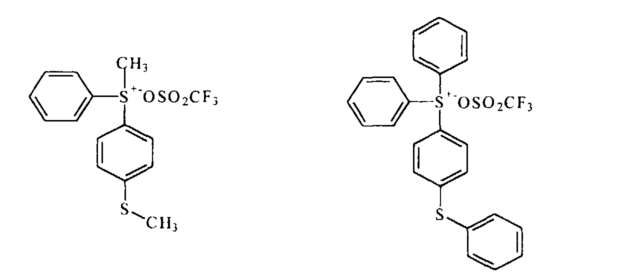

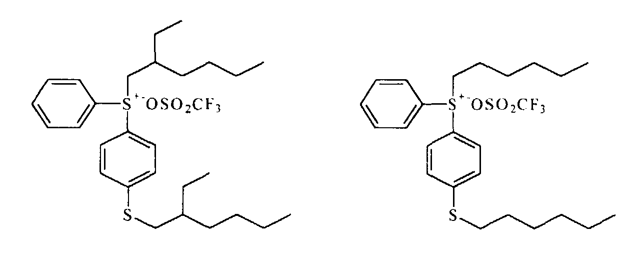

3. Process of synthesizing sulfonium salt of claim 1, wherein a sulfide compound represented by the following formula 2 is reacted with perfluoroalkanesulfonic anhydride represented by the following formula 3. 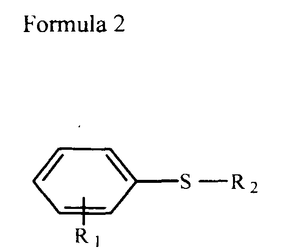
Wherein, R₁ and R₂ are same as defined above. 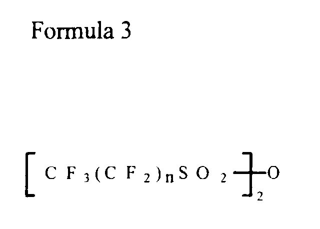
Wherein, n is same as defined above.

4. The process according to claim 3, wherein the amount of perfluoroalkanesulfonic anhydride represented by the formula 3 is used in the range of 0.2 to 2.0 equivalents to the sulfide compound represented by the formula 2.

5. The process according to claim 3, wherein the reaction is performed at the temperature of 100∼100°C.
